# FASCICULE DE BREVET EUROPEEN

(11) **EP 2 776 568 B1**
(45) Date de publication et mention de la délivrance du brevet: **18.10.2017**
(21) Numéro de dépôt: 12783528.8
(22) Date de dépôt: 06.11.2012
(51) Int. Cl.: C12N 15/85, C12N 15/861

(54) **CASSETTE D'EXPRESSION INDUCTIBLE ET SES UTILISATIONS**
KASSETTE FÜR EINE INDUZIERBARE EXPRESSION UND VERWENDUNGEN DAVON
INDUCIBLE EXPRESSION CASSETTE, AND USES THEREOF

(30) Priorité: 08.11.2011 FR 1103392
(43) Date de publication de la demande: 17.09.2014
(73) Titulaire: Institut National de la Recherche Agronomique (INRA), 75338 Paris Cedex 07 (FR); Centre National de la Recherche Scientifique (C.N.R.S.), 75794 Paris Cedex 16 (FR)
(72) Inventeur: FAFOURNOUX, Pierre, F-6320 Aurières (FR); BRUHAT, Alain, F-63450 Chanonat (FR); JOUSSE, Céline, F-63450 Saint Amant Tallende (FR); MAURIN, Anne-Catherine, F-63170 Aubière (FR); AVEROUS, Julien, F-63000 Clermont-Ferrand (FR)
(74) Mandataire: Barbot, Willy
(86) Numéro de dépôt international: PCT/EP2012/004610
(87) Numéro de publication internationale: WO 2013/068096

(56) Documents cités:
- WO-A2-01/48182
- A. BRUHAT ET AL: "Amino Acids Control Mammalian Gene Transcription: Activating Transcription Factor 2 Is Essential for the Amino Acid Responsiveness of the CHOP Promoter", MOLECULAR AND CELLULAR BIOLOGY, vol. 20, no. 19, 1 octobre 2000 (2000-10-01), pages 7192-7204, XP055029005, ISSN: 0270-7306, DOI: 10.1128/MCB.20.19.7192-7204.2000
- A. BRUHAT: "Differences in the Molecular Mechanisms Involved in the Transcriptional Activation of the CHOP and Asparagine Synthetase Genes in Response to Amino Acid Deprivation or Activation of the Unfolded Protein Response", JOURNAL OF BIOLOGICAL CHEMISTRY, vol. 277, no. 50, 6 décembre 2002 (2002-12-06), pages 48107-48114, XP055029364, ISSN: 0021-9258, DOI: 10.1074/jbc.M206149200
- Passe: "The murine p8 gene promoter is activated by activating transcription factor 4 (ATF4) in the gonadotrope-derived LbetaT2 cell line. 10.1385/ENDO", Endocrine, 1 janvier 2006 (2006-01-01), XP055057742, Extrait de l'Internet: URL:http://download.springer.com/static/pd f/787/art%3A10.1385%2FENDO%3A30%3A1%3A81.p df?auth66=1365523022_674a27080af6a2588cbf3 eefa5e3676a&ext=.pdf [extrait le 2013-03-25]

## Description

### DOMAINE DE L'INVENTION

La présente invention concerne une cassette d'expression inductible comprenant une séquence régulatrice CARE (C/EBP-ATF Responsive Element) contenant une séquence AARE (Amino Acid Response Element), un vecteur la comprenant, une composition pharmaceutique la comprenant et une combinaison comprenant ladite cassettepour leur utilisation dans le cadre de la thérapie génique.

### ART ANTERIEUR

La thérapie génique est une stratégie thérapeutique reposant sur le transfert de gène(s) dans une cellule ou un organisme hôte. Le principe apparait dès la fin des années 1960, et le premier essai clinique est initié aux Etats-Unis vingt ans après. Le concept, au préalable envisagé dans le cadre de maladies génétiques, est rapidement étendu au traitement d'un grand nombre d'autres pathologies, tels que les cancers, les maladies infectieuses ou encore les maladies cardiovasculaires.

Le but est de délivrer des gènes médicaments au patient, la plupart des stratégies envisagées utilisant des vecteurs pour véhiculer le gène thérapeutique vers sa cible cellulaire. Les premiers vecteurs développés sont basés sur une expression constitutive du gène médicament. Or, dans le cadre d'une thérapie ciblée et non toxique, il a vite paru préférable de limiter l'expression du gène thérapeutique à des types cellulaires donnés, à un temps donné, et/ou pendant une durée donnée. L'utilisation de systèmes inductibles a donc été envisagée. Ainsi, la demande internationale WO 01/48182 A2 divulgue des systèmes d'expression synthétiques comprenant un promoteur minimal associé à un élément de réponse inductible par des acides aminés tels que la sérine ou la thréonine. Par ailleurs, les travaux de Bruhat et al. (Mol. Cell. Biol. 2000, vol.20(19), pp. 7192-7204) ont permis l'identification de séquence régulatrice inductible par une carence en acide aminé et appelée AARE (Amino Acid Response Element). Même si de nombreux systèmes inductibles sont aujourd'hui à disposition de l'homme du métier, le besoin de nouveaux systèmes plus spécifiques, facilement activables et contrôlables, avec un niveau d'expression basal faible, pour un minimum d'effets secondaires, reste évident.

### SOMMAIRE DE L'INVENTION

L'invention concerne une cassette d'expression comprenant un gène d'intérêt lié de manière opérationnelle à un promoteur inductiblecomprenant (i) au moins une séquence régulatrice CARE (C/EBP-ATF responsive élément) contenant une séquence AARE (Amino Acid Response Element) et sélectionnée dans le groupe des séquences SEQ ID NO : 2, 4, 5 ou 6 et (ii) un promoteur minimal de la thymidine kinase défini par la séquence SEQ ID NO:1 pour son utilisation dans le traitement des maladies prolifératives, des maladies infectieuses, des maladies génétiques, des maladies neurologiques ou des maladies cardiovasculaires par thérapie génique..

L'invention concerne par ailleurs un vecteur comprenant une cassette d'expression comprenant un gène d'intérêt lié de manière opérationnelle à un promoteur inductible comprenant (i) au moins une séquence régulatrice CARE (C/EBP-ATF responsive élément) contenant une séquence AARE (Amino Acid Response Element) et sélectionnée dans le groupe des séquences SEQ ID NO : 2, 4, 5 ou 6 et (ii) un promoteur minimal de la thymidine kinase défini par la séquence SEQ ID NO : 1 pour son utilisation dans le traitement des maladies prolifératives, des maladies infectieuses, des maladies génétiques, des maladies neurologiques ou des maladies cardiovasculaires par thérapie génique.

L'invention est relative à une composition pharmaceutique comprenant une cassette d'expression comprenant un gène d'intérêt lié de manière opérationnelle à un promoteur inductible comprenant ou consistant en la séquence SEQ ID NO : 7 pour son utilisation dans le traitement des maladies prolifératives, des maladies infectieuses, des maladies génétiques, des maladies neurologiques ou des maladies cardiovasculaires par thérapie génique.

La demande divulgue également une méthode pour le traitement des maladies par thérapie génique, ladite méthode comprenant :
(i) une étape d'administration d'une cassette d'expression, d'un vecteur, d'une cellule hôte ou d'une composition pharmaceutique selon l'invention à un patient, et
(ii) une étape d'induction de l'expression du gène d'intérêt compris dans ladite cassette d'expression, ledit vecteur, ladite cellule hôte ou ladite composition pharmaceutique.

Plus particulièrement, ladite induction est mise en oeuvre par l'application d'un régime carencé en acide aminé, préférentiellement en un acide aminé essentiel, audit patient.

La présente invention couvre enfin une combinaison comprenant:
- une cassette d'expression comprenant un gène d'intérêt codant pour un polypeptide ayant un effet thérapeutique pour une affection ciblée lié de manière opérationnelle à un promoteur inductible comprenant (i) au moins une séquence régulatrice CARE (C/EBP-ATF responsive element) contenant une séquence AARE (Amino Acid Response Element) et (ii) un promoteur minimal, un vecteur comprenant ladite cassette d'expression, une cellule hôte comprenant ledit vecteur ou ladite cassette d'expression ou une composition pharmaceutique comprenant ladite cassette d'expression, ledit vecteur ou ladite cellule hôte, et
- un régime carencé en un acide aminé essentiel,
pour une utilisation simultanée, séparée ou séquentielle pour le traitement par thérapie génique d'une affection ciblée choisie dans le groupe comprenant les maladies prolifératives, les maladies infectieuses, les maladies génétiques, les maladies neurologiques ou les maladies cardiovasculaires.

### DESCRIPTION DES DESSINS

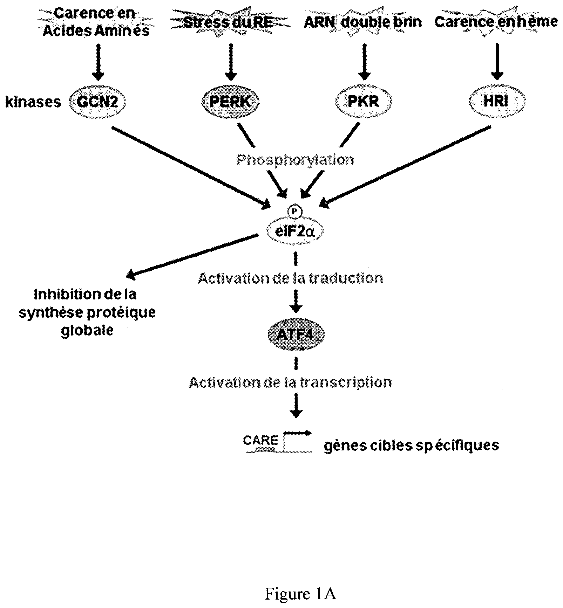

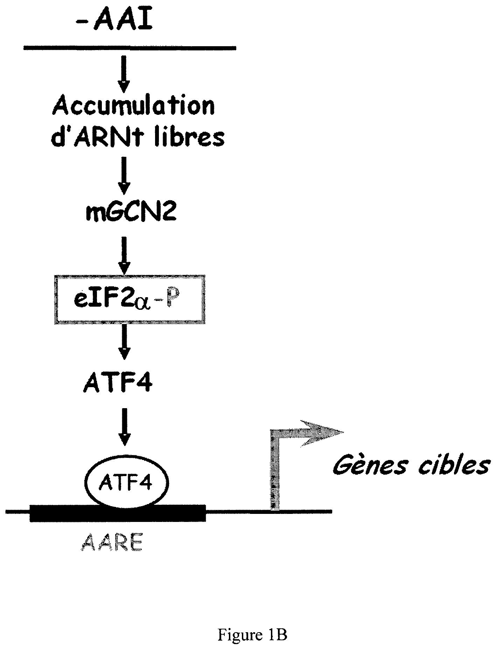

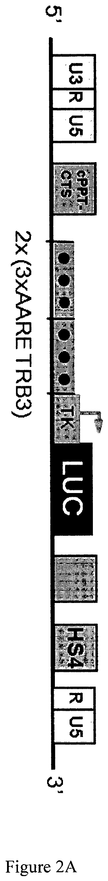

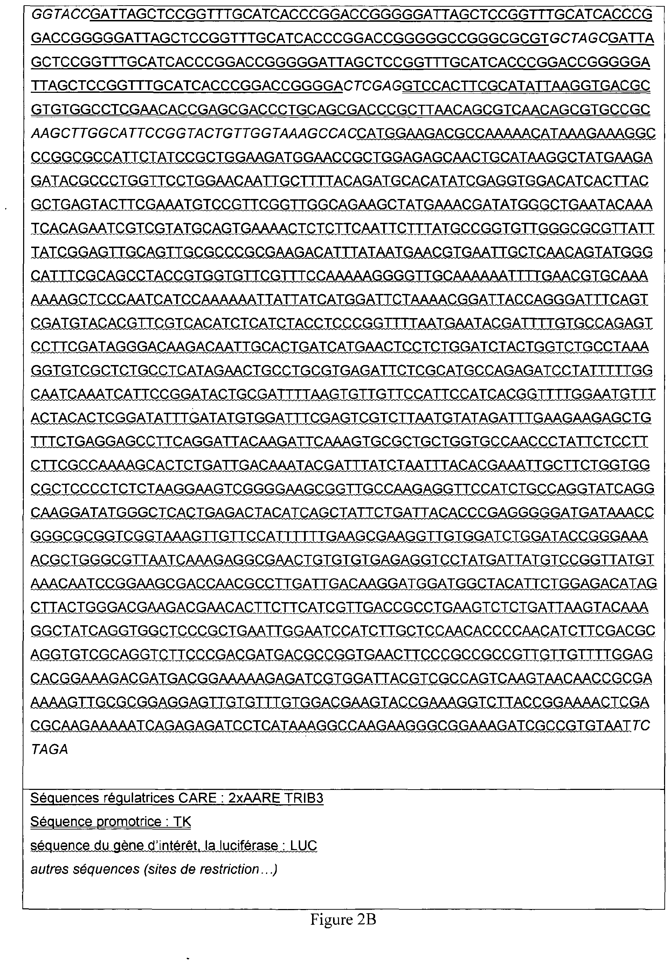

La Figure 1 décrit de manière schématique la voie de signalisation eIF2α/ATF4, et notamment la voie GCN2/ATF4.
La figure 1A décrit la voie de signalisation générale.
La figure 1B décrit plus spécifiquement la voie de signalisation GCN2/ATF4. La carence en n'importe quel acide aminé essentiel (ou indispensable) active la kinase GCN2. Suite à la phosphorylation eIF2α, le facteur de transcription ATF4 est induit. Il va se fixer sur les éléments AARE localisés dans le promoteur de gènes cibles et activer la transcription.
La Figure 2A illustre un Schéma de la construction 2XAARE-TK-Luc (utilisée dans la partie expérimentale) insérée dans un vecteur d'expression rétroviral.
La Figure 2B représente la séquence du transgène 2XAARE-TK-LUC utilisée dans la partie expérimentale.

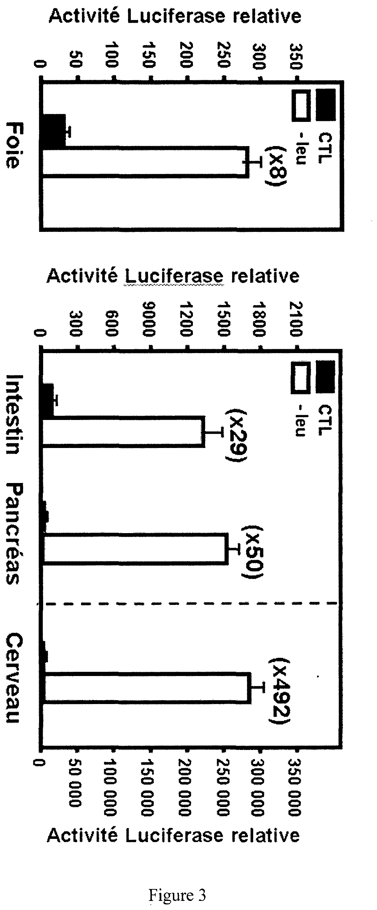

La Figure 3 représente le dosage de l'activité Luciférase dans les tissus des souris transgéniques arborant un transgène comprenant un vecteur selon l'invention avec la luciférase comme gène l'intérêt, deux copies de la séquence CARE du gène TRIB3 (séquences AARE) et le promoteur minimum de la thymidine kinase (souris AARE-LUC). Au début de l'expérience nutritionnelle, les souris CARE-LUC ont été mises à jeun pendant 16h. Le jour de l'expérience, les souris ont été nourries pendant 4h, soit avec le régime contrôle (CTL) ou soit avec un régime carencé en leucine (-leu). Après sacrifice des souris, l'activité luciférase a été dosée dans le foie, l'intestin, le pancréas et le cerveau.

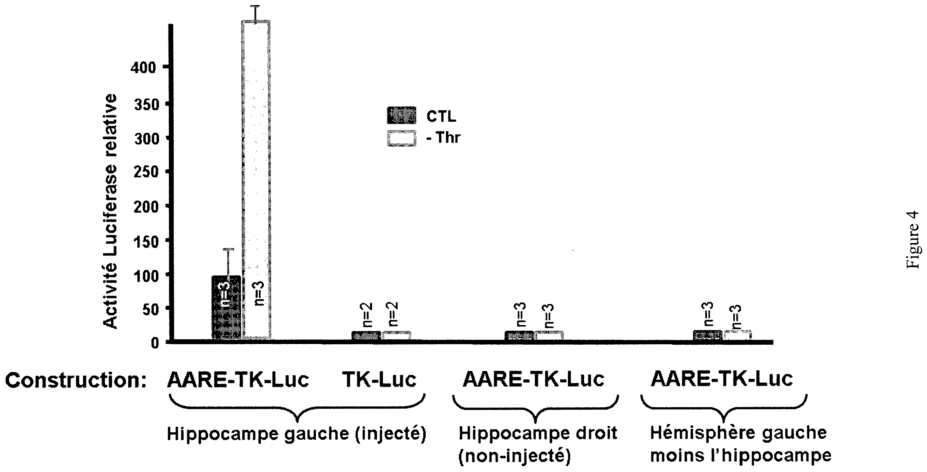

La Figure 4 représente la mesure de l'activité luciférase après injection de lentivirus dans le cerveau.
   Les vecteurs lentiviraux sont injectés dans l'hippocampe de l'hémisphère gauche des rats. Deux semaines après injection les animaux sont mis à jeun une nuit puis nourris avec un repas contrôle ou carencé en Thréonine (-Thr). Six heures après le début du repas les animaux sont sacrifiés, les cerveaux prélevés et disséqués. L'activité luciférase est mesurée dans les hippocampes droits et gauches ainsi que le reste de l'hémisphère gauche. L'activité Luc et ensuite normalisée par rapport au contenu protéique. Deux constructions ont été utilisées: la construction 2XAARE-TK-LUC (décrite dans la figure 2) et la construction contrôle TK-LUC où les séquences AARE ont été enlevées.

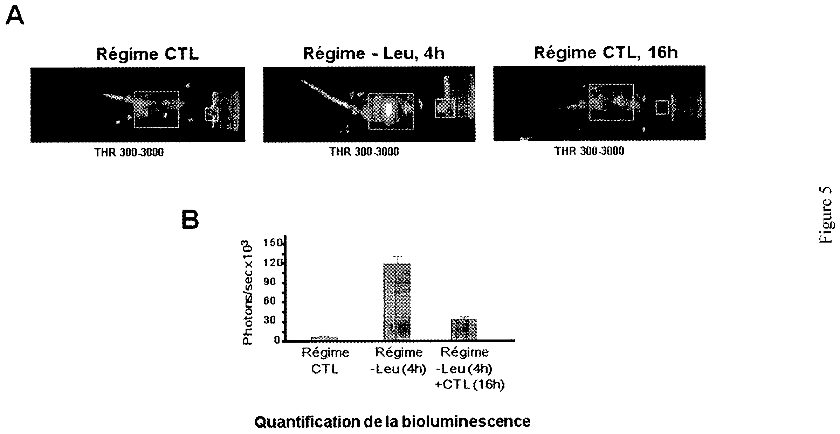

La Figure 5 montre la réversibilité de l'induction du transgène AARE-LUC chez la souris suite à la consommation d'un régime carencé en leucine. (A) Visualisation de l'activité luciférase par imagerie en bioluminescence (seuil de détection 300-3000) sur une souris nourrie successivement avec (1) un régime contrôle (CTL), (2) un régime carencé en leucine (-Leu) pendant 4h, et (3) un régime CTL pendant 16h. (B) Quantification de la bioluminescence dans les zones encadrées. La luciférase utilisée possède une demi-vie longue ce qui explique les 16h nécessaires pour réduire de façon significative l'activité luciférase.

### DESCRIPTION DETAILLEE DE L'INVENTION

La présente invention a pour objet une cassette d'expression comprenant un gène d'intérêt lié de manière opérationnelle à un promoteur inductible comprenant (i) au moins une séquence régulatrice CARE (C/EBP-ATF Responsive Element) contenant une séquence AARE (Amino Acid Response Element) et sélectionnée dans le groupe des séquences SEQ ID NO : 2, 4, 5 ou 6 et (ii) un promoteur minimal de la thymidine kinase défini par la séquence SEQ ID NO :1 pour son utilisation dans le traitement des maladies prolifératives, des maladies infectieuses, des maladies génétiques, des maladies neurologiques ou des maladies cardiovasculaires par thérapie génique.

Le terme « cassette d'expression » fait référence à un élément comprenant des séquences d'acides nucléiques spécifiques à intégrer dans une autre molécule d'acide nucléique ou un génome. Une cassette d'expression comprend en général un ou plusieurs gènes ainsi que les éléments de contrôle de l'expression dudit (desdits) gène(s) (promoteur, terminateur...).

Le terme « promoteur » est bien connu de l'homme de l'art et se réfère à une région de l'ADN située à proximité d'un gène sur laquelle se fixe l'ARN polymérase, afin de démarrer la transcription.

Généralement, l'expression « lié de manière opérationnelle » signifie que la séquence promotrice est positionnée par rapport à la séquence codante du gène d'intérêt de manière à ce que la transcription puisse commencer. Cela signifie que le promoteur est positionné en amont de la région codante, à une distance permettant l'expression de cette dernière.

Il existe différents types de promoteurs tels que les promoteurs constitutifs ou les promoteurs inductibles. Les promoteurs inductibles sont des promoteurs dont l'activité est contrôlée par des conditions environnementales particulières ou la présence d'un composé particulier ; ils permettent donc un contrôle de l'expression du gène d'intérêt.

Les promoteurs utilisés dans le cadre de l'invention peuvent être dérivés de gènes natifs, ou composés de différents éléments dérivés de différents promoteurs naturels, ou encore peuvent comprendre des segments d'ADN synthétiques.

On entend par « promoteur minimal » un promoteur consistant en un site d'initiation de la transcription et des séquences pour la fixation du complexe d'initiation de la transcription (TATA-box) fonctionnels dans une cellule ou un organisme hôte.

Ces éléments sont classiques dans le domaine de l'art concerné. On peut citer plus particulièrement les promoteurs minimaux des gènes TK, CMV et HSP (promoteur minimal du gène de protéine de choc thermique de drosophile dépourvu de sa séquence activatrice).

De préférence, le promoteur utilisé selon l'invention est le promoteur minimal de la thymidine kinase ou un dérivé de celui-ci. Le promoteur minimal de la thymidine kinase correspond à la partie -40 à +50 de la séquence de la thymidine kinase, +1 étant le site d'initiation de la transcription (Majumder S, DePamphilis ML, Mol Cell Biol, 1994) ; il est défini par la séquence SEQ ID NO :1.

| | |
|---|---|
| Séquence du promoteur minimal de la thymidine kinase (SEQ ID NO :1) | |

Le terme "dérivé de" correspond à une séquence d'acide nucléique ayant au moins 90% d'identité avec une séquence de référence, particulièrement au moins 95% d'identité et préférentiellement au moins 99% d'identité. Le terme « pourcentage d'identité entre deux séquences d'acide nucléique » fait référence au pourcentage de nucléotides identiques entre deux séquences comparées, ledit pourcentage étant obtenu par le meilleur alignement de la séquence entière. Le terme « meilleur alignement » correspond à l'alignement qui permet d'obtenir le pourcentage d'identité le plus élevé. Il peut être obtenu en utilisant différents algorithmes bien connus de l'homme du métier (GAP, BESTFIT, BLAST P, BLAST N, FASTA, TFASTA, GENETICS COMPUTER GROUP, 575 Science Dr., Madison, WI USA).

Le promoteur peut être un promoteur eucaryote qui doit être fonctionnel dans l'organisme hôte.

Par « séquence régulatrice CARE » (C/EBP-ATF responsive élément), on entend toute séquence cible de la voie de signalisation eIF2α/ATF4 capable de lier le facteur de transcription ATF4. Dans cette voie, le facteur de transcription ATF4 (activating transcription factor 4, aussi appelé CREB2, TAXREB67, TXREB) se lie à ces séquences CARE pour induire ou réguler la transcription de gènes cibles en réponse à un stress environnemental donné (Kilberg et al., Trends Endocrinol Metab. 2009 Nov;20(9):436-43).

Différentes séquences CARE ont été décrites, notamment pour les gènes suivants : CHOP (C/EBP homologous protein, aussi appelé GADD153), ASNS (Asparagine Synthetase), SNAT2 (System A Amino Acid Transporter), ATF3, TRIB3, Cat-1, xCT, HERP, VEGF et 4E-BP1.

Plus particulièrement, dans le cadre de l'invention, on entend par « Amino Acid Response Element » (AARE) des séquences régulatrices CARE ciblées par le facteur de transcription ATF4 en cas de carence en acide aminé : ces séquences AARE sont des séquences CARE particulières ciblées en cas de phosphorylation d'eIF2α par la kinase GCN2, cette phosphorylation étant induite par une carence en un acide aminé, préférentiellement une carence en un acide aminé essentiel.

Des exemples de séquences AARE utilisées selon l'invention comprennent, mais ne sont pas limitées aux séquences CARE des gènes ASNS, SNAT2, ATF3 et TRIB3.

Dans un mode particulier de réalisation de l'invention, la séquence régulatrice CARE contenant une séquence AARE présente dans la cassette d'expression utilisée selon l'invention comprend ou consiste en une séquence sélectionnée parmi le groupe comprenant : la séquence CARE du gène TRIB3 (SEQ ID NO :2), la séquence CARE du gène ASNS (SEQ ID NO :4), la séquence CARE du gène ATF3 (SEQ ID NO :5) et la séquence CARE du gène SNAT2 (SEQ ID NO :6), ou un dérivé de celle-ci.

De préférence, ladite séquence régulatrice CARE contenant une séquence AARE présente dans la cassette d'expression utilisée selon l'invention comprend ou consiste en une ou plusieurs copies de la séquence SEQ ID NO :2, ou un dérivé de celle-ci.

| | |
|---|---|
| Séquence CARE du gène TRIB3 (SEQ ID NO :2) | CGGTTTGCATCACCCG |
| Séquence CARE du gène CHOP (SEQ ID NO :3) | AACATTGCATCATCCC |
| Séquence CARE du gène ASNS (SEQ ID NO :4) | GAAGTTTCATCATGCC |
| Séquence CARE du gène ATF3 (SEQ ID NO :5) | AGCGTTGCATCACCCC |
| Séquence CARE du gène SNAT2 (SEQ ID NO :6) | GATATTGCATCAGTTT |

Dans un mode de réalisation particulier de l'invention, le promoteur inductible compris dans la cassette d'expression utilisée selon l'invention comprend une ou plusieurs copies d'une séquence régulatrice CARE contenant une séquence AARE (Amino Acid Response Element). Plus particulièrement, ledit promoteur comprend au moins une, au moins deux ou au moins trois copies d'une séquence régulatrice CARE contenant une séquence AARE (Amino Acid Response Element).

Plus particulièrement, le promoteur inductible compris dans la cassette d'expression utilisée selon l'invention comprend deux copies d'une séquence CARE contenant une séquence AARE (Amino Acid Response Element).

De préférence, le promoteur inductible compris dans la cassette d'expression utilisée selon l'invention comprend deux copies de la séquence CARE contenant une séquence AARE (Amino Acid Response Element) de TRIB3 (SEQ ID NO :2).

Dans un mode de réalisation préféré, le promoteur de la cassette d'expression utilisée selon l'invention comprend deux copies de la séquence CARE contenant une séquence AARE (Amino Acid Response Element)du gène TRIB3 (SEQ ID NO :2) et le promoteur de la thymidine kinase (SEQ ID NO :1). Préférentiellement, le promoteur de la cassette d'expression utilisée selon l'invention comprend ou consiste en la séquence SEQ ID NO :7 ou un dérivé de celle-ci.

| | |
|---|---|
| SEQ ID NO:7 | |

Selon l'invention, la séquence régulatrice CARE contenant une séquence AARE (Amino Acid Response Element) comprise dans la cassette d'expression utilisée selon l'invention est positionnée en amont (en position 5') ou en aval (en position 3') du promoteur minimal. Préférentiellement, elle est positionnée en amont.

Le promoteur inductible utilisé dans l'invention, comprenant au moins une séquence régulatrice CARE (C/EBP-ATF Responsive Element) contenant une séquence AARE (Amino Acid Response Element) et un promoteur minimal, est inductible de préférence par une carence en acide aminé (en un ou plusieurs acide(s) aminé(s)), plus préférentiellement par une carence en acide aminé essentiel.

Le gène d'intérêt en usage dans la présente invention, peut être issu d'un organisme eucaryote, d'un procaryote, d'un parasite ou d'un virus. Il peut être isolé par toute technique conventionnelle dans le domaine de l'art, par exemple par clonage, PCR ou synthèse chimique. Il peut être de type génomique, de type ADN complémentaire (ADNc) ou de type mixte (minigène). Par ailleurs, il peut coder pour un ARN antisens ou un ARN interférent (tel que des micro-ARN (ou miRNApour microRNA), des siRNA (small interfering RNA), des dsRNA (double strand RNA), des shRNA (short RNA)...), et/ou un ARN messager (ARNm) qui sera ensuite traduit en polypeptide d'intérêt celui-ci pouvant être intracellulaire, incorporé dans la membrane de la cellule hôte ou encore secrété. Il peut s'agir d'un polypeptide tel que trouvé dans la nature, d'une portion de celui-ci, d'un mutant présentant notamment des propriétés biologiques améliorées ou modifiées ou encore d'un polypeptide chimère provenant de la fusion de séquences d'origines diverses. Par ailleurs, le gène d'intérêt peut coder pour un ARN anti-sens, un ribozyme, ou encore un polypeptide d'intérêt.

La cassette d'expression utilisée selon l'invention permettra d'exprimer, de surexprimer ou d'inhiber l'expression d'un gène d'intérêt. Cette modulation de l'expression du gène d'intérêt pourra de préférence être contrôlée par l'application ou non d'un régime carencé en acide(s) aminé(s), préférentiellement en acide(s) aminé(s) essentiel(s).

Parmi les polypeptides d'intérêt utilisables, on peut citer plus particulièrement les chémokines et cytokines (interféron α, β ou y, interleukine (IL), notamment l'IL-2, l'IL-6, l'IL-10 ou encore l'IL-12, facteur nécrosant des tumeurs (TNF), facteur stimulateur de colonies (GM- CSF, C-CSF, M-CSF... ), MIP-1α, MIP-1β, RANTES, protéine de chémoattraction des monocytes telle que MCP-1...), les récepteurs cellulaires (notamment reconnus par le virus HIV), les ligands de récepteur, les facteurs de coagulation (Facteur VIII, Facteur IX, facteur X, thrombine, protéine C...), les facteurs de croissance (FGF (Fibroblast Growth Factor), VEGF (Vascular Endothelial Growth Factor)), les enzymes (kinases, phosphatases, uréase, rénine, métalloprotéinase, NOS (nitric oxide synthétase), SOD, catalase, LCAT (lécithine cholestérol acyl transférase)...), les inhibiteurs d'enzyme (OE1- antitrypsine, antithrombine HI, inhibiteur de protéase virale, PAI-1 (plasminogen activator inhibitor)), les antigènes du complexe majeur d'histocompatibilité de classe I ou II ou polypeptides agissant sur l'expression des gènes correspondants, les polypeptides capables d'inhiber une infection virale, bactérienne ou parasitaire ou son développement, les polypeptides agissant positivement ou négativement sur l'apoptose (Bax, Bcl2, BclX...), les agents cytostatiques (p21, p16, Rb), les immunoglobulines en totalité ou en partie (Fab, ScFv...), les toxines, les immunotoxines, les apolipoprotéines (ApoAI, ApoAIV, ApoE...), les inhibiteurs d'angiogenèse (angiostatine, endostatine...), les marqueurs (β-galactosidase, luciférase...) ou tout autre polypeptide ayant un effet thérapeutique pour l'affection ciblée.

Plus précisément, dans le but de traiter un dysfonctionnement héréditaire, on utilisera une copie fonctionnelle du gène défectueux, par exemple un gène codant pour le facteur VIII ou IX dans le cadre de l'hémophilie A ou B, la dystrophine (ou minidystrophine) dans le cadre des myopathies de Duchenne et Becker, l'insuline dans le cadre du diabète, la protéine CFTR (Cystic Fibrosis Transmembrane Conductance Regulator) dans le cadre de la mucoviscidose.

S'agissant d'inhiber l'initiation ou la progression de tumeurs ou cancers, on mettra de préférence en oeuvre un gène d'intérêt codant pour un ARN anti-sens ou un ARN interférent (miRNA, siRNA, dsRNA, shRNA...), un ribozyme, un produit cytotoxique (comme la thymidine kinase de virus simplex de l'herpès 1 (TK-HSV-1), la ricine, les toxines cholérique et diphtérique, un produit des gènes de levure FCY1 et FUR1 codant pour l'uracyle phosphoribosyl transférase ou encore la cytosine désaminase), une immunoglobuline, un inhibiteur de la division cellulaire ou des signaux de transduction, un produit d'expression d'un gène suppresseur de tumeur (p53, Rb, p73, DCC...), un polypeptide stimulateur du système immunitaire, un antigène associé à une tumeur (MUC-1, BRCA-1, des antigènes précoces ou tardifs (E6, E7, LI, L2...) d'un papillomavirus humain HPV...), éventuellement en combinaison avec un gène de cytokine.

Un exemple de gène d'intérêt permettant d'inhiber l'expression d'une protéine serait un shRNA dirigé contre le récepteur beta2ACh, qui pourrait être utilisé pour réduire l'addiction au tabac (Maskos et al., Nature 2005).

Enfin, dans le cadre d'une thérapie anti-HIV, on peut avoir recours à un gène codant pour un polypeptide immunoprotecteur, un épitope antigénique, un anticorps, le domaine extracellulaire du récepteur CD4 (sCD4 ; Traunecker et al., 1988, Nature 331, 84-86) une immunoadhésine (par exemple un hybride CD4- immunoglobuline IgG , Capon et al., 1989, Nature 33 7, 525-531 ; Byrn et al., 1990, Nature 344, 667-670), une immunotoxine (par exemple fusion de l'anticorps 2F5 ou de l'immunoadhésine CD4-2F5 à l'angiogénine; Kurachi et aL, 1985, Biochemistry 24, 5494-5499), un variant trans-dominant (EP 0614980, WO95/16780), un produit cytotoxique tel que l'un de ceux mentionné ci-dessus ou encore un IFNα ou β.

Un des gènes d'intérêt peut également être un gène de sélection permettant de sélectionner ou identifier les cellules transfectées ou transduites. On peut citer les gènes néo (codant pour la néomycine phosphotransférase) conférant une résistance à l'antibiotique G418, dhfr (Dihydrofolate Réductase), CAT (Chloramphenicol Acetyl Transférase), pac (Puromycine Acétyl-Transférase) ou encore gpt (Xanthine Guanine Phosphoribosyl Transférase). D'une manière générale, les gènes de sélection sont connus de l'homme de l'art.

Par ailleurs, la cassette d'expression utilisée selon l'invention peut, en outre, inclure des éléments additionnels améliorant son expression ou son maintien dans la cellule hôte (origines de réplication, éléments d'intégration dans le génome cellulaire, séquences introniques, séquences poly A de terminaison de la transcription, leaders tripartites...). Ces éléments sont connus de l'homme de l'art.

En outre, le gène d'intérêt peut également comporter en amont de la région codante une séquence codant pour un peptide signal permettant sa sécrétion de la cellule hôte. Le peptide signal peut être celui du gène en question ou hétérologue (issu d'un gène quelconque sécrété ou synthétique).

Enfin, le gène d'intérêt contient un terminateur.

Dans un mode de réalisation particulier de l'invention, la cassette d'expression utilisée selon l'invention comprend en outre la séquence 5'UTR du gène ATF4 (SEQ ID NO :8, Vattem et al, PNAS, 2004 ; Lu et al J.Cell. Biol. 2004 ) ou une séquence similaire, en amont du site d'initiation de la traduction de la cassette.

Selon l'invention, une séquence similaire signifie une séquence 5' UTR d'un gène ayant la même régulation traductionnelle qu'ATF4. Des exemples de gènes ayant une séquence 5' UTR similaire comprennent, mais ne sont pas limités à : GADD34 (Lee et al, J Biol Chem, 2009), ATF5 (Zhou et al, J Biol Chem, 2008), BACE1 (Zhou et al, Mol Cell Biol, 2006).

| | |
|---|---|
| SEQ ID NO:8 | |

L'invention a par ailleurs pour objet un vecteur comprenant une cassette d'expression comprenant un gène d'intérêt lié de manière opérationnelle à un promoteur inductible comprenant (i) au moins une séquence régulatrice CARE (C/EBP-ATF responsive élément) contenant une séquence AARE (Amino Acid Response Element) et sélectionnée dans le groupe des séquences SEQ ID NO: 2, 4, 5 ou 6 et (ii) un promoteur minimal de la thymidine kinase défini par la séquence SEQ ID NO : 1 pour son utilisation dans le traitement des maladies prolifératives, des maladies infectieuses, des maladies génétiques, des maladies neurologiques ou des maladies cardiovasculaires par thérapie génique.

Selon l'invention, il peut s'agir d'un vecteur synthétique (lipides cationiques, liposomes polymères...), d'un plasmide ou encore d'un vecteur viral.

Il peut être éventuellement associé à une ou plusieurs substances améliorant l'efficacité transfectionnelle et/ou la stabilité du vecteur. Ces substances sont largement documentées dans la littérature accessible à l'homme de l'art (voir par exemple Felgner et al., 1987, Proc. West. Pharmacol. Soc. 32, 115- 121; Hodgson et Solaiman, 1996, Nature Biotechnology 14, 339-342 ; Remy et al. , 1994, Bioconjugate Chemistry 5, 647-654). A titre illustratif mais non limitatif, il peut s'agir de polymères, de lipides notamment cationiques, de liposomes, de protéines nucléaires ou encore de lipides neutres. Ces substances peuvent être utilisées seules ou en combinaison. Une combinaison envisageable est un vecteur recombinant plasmidique associé à des lipides cationiques (DOGS, DC-CHOL, spermine-chol, spermidine-chol etc.) et des lipides neutres (DOPE).

Le choix des plasmides utilisables dans le cadre de la présente invention est vaste. Il peut s'agir de vecteurs de clonage et/ou d'expression. D'une manière générale, ils sont connus de l'homme de l'art et nombre d'entre eux sont disponibles commercialement mais il est également possible de les construire ou les modifier par les techniques de manipulation génétique. On peut citer à titre d'exemples les plasmides dérivés de pBR322 (Gibco BRL), pUC (Gibco BRL), pBluescript (Stratagène), pREP4, pCEP4 (Invitrogene) ou encore p Poly (Lathe et al., 1987, Gene 57, 193-201). De préférence, un plasmide mis en oeuvre dans le cadre de la présente invention contient une origine de réplication assurant l'initiation de la réplication dans une cellule productrice et/ou une cellule hôte (par exemple, on retiendra l'origine CoIEI pour un plasmide destiné à être produit dans E. coli et le système oriP/EBNAI si l'on désire qu'il soit auto réplicatif dans une cellule hôte mammifère, Lupton et Levine, 1985, Mol. Cell. Biol. 5, 2533- 2542 ; Yates et al., Nature 313, 812-815). Il peut en outre comprendre un gène de sélection permettant de sélectionner ou identifier les cellules transfectées (complémentation d'une mutation d'auxotrophie, gène codant pour la résistance à un antibiotique...). Il peut aussi comprendre des éléments supplémentaires améliorant son maintien et/ou sa stabilité dans une cellule donnée (séquence cer qui favorise le maintien monomérique d'un plasmide, séquences d'intégration dans le génome cellulaire).

S'agissant d'un vecteur viral, on peut envisager un vecteur dérivant d'un adénovirus, d'un lentivirus, d'un rétrovirus, d'un virus associé à l'adénovirus (AAV), d'un virus de l'herpès, d'un alphavirus, d'un parvovirus, d'un poxvirus (fowlpox, canaripox, virus de la vaccine notamment de la souche MVA (Modified Virus Ankara) ou Copenhagen...) ou d'un foamyvirus. On aura de préférence recours à un vecteur non réplicatif et, éventuellement, non intégratif. Les rétrovirus ont la propriété d'infecter et de s'intégrer majoritairement dans les cellules en division et à cet égard sont particulièrement appropriés pour une application en thérapie anticancéreuse. Un vecteur rétroviral convenant à la mise en oeuvre de la présente invention comporte les séquences terminales LTR (Long Terminal Repeat) et une région d'encapsidation. Il peut dériver d'un rétrovirus d'une origine quelconque (murin, primate, félin, humain etc.) et en particulier peut dériver d'un rétrovirus choisi parmi le groupe comprenant MoMuLV (Moloney murine leukernia virus), MVS (Mûrine sarcoma virus) ou Friend murine rétrovirus (Fb29). Il est propagé dans une lignée d'encapsidation capable de fournir en trans les polypeptides viraux gag, pol et/ou env nécessaires à la constitution d'une particule virale. De telles lignées sont décrites dans la littérature (PA317, Psi CRIP GP + Am-12 etc.). Le vecteur rétroviral selon l'invention peut comporter des modifications notamment au niveau des LTRs (remplacement de la région promotrice par un promoteur eucaryote) ou de la région d'encapsidation (remplacement par une région d'encapsidation hétérologue, par exemple de type VL30) (voir les demandes françaises 94 08300 et 97 05203).

Dans un mode de réalisation préféré de l'invention, le vecteur utilisé selon l'invention est un vecteur lentiviral, un vecteur adénoviral ou un vecteur dérivé d'un virus associé à l'adénovirus (AAV).

Selon un mode de réalisation avantageux, le vecteur viral utilisé selon l'invention peut être sous la forme de vecteur ADN ou de particule virale infectieuse.

La présente demande divulgue également une cellule hôte comprenant un vecteur ou une cassette d'expression pour une utilisation selon l'invention.

Telle qu'utilisée ici, une cellule est constituée par toute cellule transfectable par un vecteur tel que décrit ci-avant.

En particulier, une cellule de mammifère, préférentiellement une cellule humaine, peut être utilisée. Il peut s'agir d'une cellule primaire ou tumorale d'une origine quelconque, notamment hématopoïétique (cellule souche totipotente, leucocyte, lymphocyte, monocyte ou macrophage...), musculaire (cellule satellite, myocyte, myoblaste, muscle lisse...), cardiaque, pulmonaire, trachéale, hépatique, épithéliale ou fibroblaste, mais aussi les cellules souches.

La présente invention a également pour objet une composition pharmaceutique comprenant une cassette d'expression comprenant un gène d'intérêt lié de manière opérationnelle à un promoteur inductible comprenant ou consistant en la séquence SEQ ID NO : 7 pour son utilisation dans le traitement des maladies prolifératives, des maladies infectieuses, des maladies génétiques, des maladies neurologiques ou des maladies cardiovasculaires par thérapie génique.

Une composition pharmaceutique utilisée selon l'invention est plus particulièrement destinée au traitement préventif ou curatif de maladies par thérapie génique (y compris immunothérapie) et s'adresse plus particulièrement aux maladies prolifératives (cancers, tumeurs, dysplasies etc.), aux maladies infectieuses et notamment virales (induites par exemple par les virus de l'hépatite B ou C, le VIH (Virus de l'Immunodéficience Humaine), l'herpès, les rétrovirus etc.), aux maladies génétiques (mucoviscidose, myopathies, hémophilies, diabète...), aux maladies cardiovasculaires (resténose, ischémie, dyslipidémie...) ou encore aux maladies neurologiques (maladies psychiatriques, maladies neurodégénératives comme Parkinson ou Alzheimer, addictions, par exemple au tabac, à l'alcool, aux drogues, épilepsie...).

Une composition pharmaceutique utilisée selon l'invention peut être fabriquée de manière conventionnelle en vue d'une administration par voie locale, parentérale ou digestive, mais aussi par stéréotaxie. En particulier, on associe une quantité thérapeutiquement efficace de l'agent thérapeutique ou prophylactique à un support acceptable d'un point de vue pharmaceutique. Les voies d'administration envisageables sont multiples. On peut citer par exemple la voie intra-gastrique, sous-cutanée, intra-cardiaque, intra-musculaire, intra-veineuse, intra-artérielle, intra-péritonéale, intra-tumorale, intra-nasale, intra-pulmonaire ou intra-trachéale. Pour ces trois derniers modes de réalisation, une administration par aérosol ou instillation est avantageuse.

L'administration peut avoir lieu en dose unique ou répétée une ou plusieurs fois après un certain délai d'intervalle. La voie d'administration et les doses appropriées varient en fonction de divers paramètres, par exemple, de l'individu, de la pathologie, du gène d'intérêt à transférer, de la voie d'administration. Lorsque l'on met en oeuvre un vecteur, des doses comprenant de 0,01 à 100 mg d'ADN, de préférence 0,05 à 10 mg et, de manière tout à fait préférée, 0, 5 à 5 mg peuvent être envisagées.

La formulation peut également inclure un diluant, un adjuvant ou un excipient acceptable d'un point de vue pharmaceutique, de même que des agents de solubilisation, de stabilisation, de préservation. Une composition préférée est sous forme injectable. Elle peut être formulée en solution aqueuse, saline (phosphate, monosodique, disodique, magnésium, potassium...) ou isotonique.

Elle peut être présentée en dose unique ou en multi-doses sous forme liquide ou sèche (poudre, lyophilisat...) susceptible d'être reconstituée de manière extemporanée par un diluant approprié.

La présente demande divulgue également l'utilisation thérapeutique ou prophylactique d'une cassette d'expression, d'un vecteur ou d'une cellule hôte tels que décrits précédemment, pour la préparation d'un médicament destiné au transfert et à l'expression d'un gène d'intérêt (compris dans ladite cassette d'expression, ledit vecteur ou ladite cellule hôte) dans une cellule ou un organisme hôte. En particulier, la cassette d'expression, le vecteur ou la cellule hôte utilisés selon l'invention sont destinés au traitement du corps humain ou animal par thérapie génique.

L'invention a donc pour objet une cassette d'expression, un vecteur la comprenant tels que décrits précédemment pour l'utilisation dans le traitement des maladies par thérapie génique.

Selon une première possibilité, le médicament peut être administré directement in vivo (par exemple par injection intraveineuse, dans une tumeur accessible, dans les poumons par aérosol, dans le système vasculaire au moyen d'une sonde appropriée ou encore par stéréotaxie dans le cerveau). On peut également adopter l'approche ex vivo qui consiste à prélever des cellules du patient (cellules souches de la moelle osseuse, lymphocytes du sang périphérique, cellules musculaires...), à les transfecter in vitro selon les techniques de l'art et à les ré-administrer au patient après une étape d'amplification éventuelle.

La prévention et le traitement de nombreuses pathologies peuvent être envisagés. Une utilisation préférée consiste à traiter ou prévenir les cancers, tumeurs et maladies résultant d'une prolifération cellulaire non désirée. Parmi les applications envisageables, on peut citer les cancers du sein, de l'utérus (notamment ceux induits par les papilloma virus HPV), de la prostate, du poumon, de la vessie, du foie, du colon, du pancréas, de l'estomac, de l'oesophage, du larynx, du système nerveux central et du sang (lymphomes, leucémie etc.). Elle est également utile dans le cadre des maladies cardiovasculaires, par exemple pour inhiber ou retarder la prolifération des cellules de muscles lisses de la paroi vasculaire (resténose). En outre, pour ce qui est des maladies infectieuses, l'application au SIDA (Syndrome d'Immuno Déficience Acquise) peut notamment être envisagée. Enfin, elle particulièrement appropriée pour le traitement des maladies neurologiques (maladies psychiatriques, neurodégénératives, addictions...).

L'invention divulgue également une méthode pour le traitement des maladies par thérapie génique, ladite méthode comprenant :
(iii) une étape d'administration d'une cassette d'expression, d'un vecteur, d'une cellule hôte ou d'une composition pharmaceutique tels que décrits précédemment à un patient, et
(iv) une étape d'induction de l'expression du gène d'intérêt compris dans ladite cassette d'expression, ledit vecteur, ladite cellule hôte ou ladite composition pharmaceutique.

Le terme « patient » fait référence à un mammifère, préférentiellement un humain, étant atteint d'une pathologie pouvant être traitée par thérapie génique. Ces pathologies sont connues de l'homme du métier, et comprennent notamment les cancers, les maladies infectieuses, les maladies cardiovasculaires et les maladies génétiques. Des exemples sont décrits dans la présente description.

L'étape d'administration est réalisée selon des méthodes connues dans l'art antérieur.

L'étape d'induction est réalisée en soumettant ledit patient à un stimulus enviromemental permettant d'induire la phosphorylation d'eIF2α (et ainsi l'activation de la voie de signalisation eIF2α/ATF4).

Ainsi, le stimulus subi par le patient peut être une carence en acide aminé, de préférence une carence en acide aminé essentiel.

Dans ce cas, la cassette d'expression (comprise ou non dans un vecteur ou une cellule hôte) utilisée selon l'invention comprend plus particulièrement la séquence régulatrice CARE contenant une séquence AARE d'un gène sélectionné dans le groupe comprenant TRIB3, ASNS, ATF3 et SNAT2 ou une séquence dérivée de celles-ci. Préférentiellement, le promoteur utilisé est le promoteur défini par la séquence SEQ ID NO :7.

Le stimulus subi par le patient peut être l'induction d'une infection virale ou l'administration d'une molécule mimant une infection virale. En effet, la voie eIF2α/ATF4 est activée lors d'une infection virale par la présence d'ARN bicaténaire produit par le virus, par les cytokines et l'interféron, ou encore par la protéine PACT et par l'héparine. Ainsi, une infection virale peut être mimée par l'un de ces éléments.

Le stimulus subi par le patient peut également être l'induction d'un stress du réticulum endoplasmique. Un tel stress peut notamment être induit par un certain nombre de drogues telles que l'anticancéreux bortezomyb, la tunicamycine, le dithiothreitol, la thapsigargine ou la brefeldine A, ou par une injection de lipopolysaccharide.

Le stimulus subi par le patient peut être l'induction d'une carence en hème.

Le stimulus subi par le patient peut être un régime riche en lipides.

Le stimulus subi par le patient peut être un choc thermique ou un choc osmotique.

Dans un mode de réalisation préféré de l'invention, le patient est soumis à un régime carencé en un acide aminé essentiel (aussi appelé acide aminé indispensable). Les acides aminés essentiels sont la phénylalanine, la leucine, la méthionine, la lysine, l'isoleucine, la vanille, la thréonine, le tryptophane et l'histidine.

Par "régime carencé en acide aminé", on entend tout moyen ou toute composition permettant d'appliquer à un patient une carence en acide aminé telle que décrite ci-dessus. De préférence, on applique au patient une carence en un acide aminé essentiel. Il peut s'agir de compositions comprenant un cocktail d'acides aminés libres dans lequel un acide aminé, préférentiellement un acide aminé essentiel, est absent.

Un tel régime sera préférentiellement administré après un jeune de courte durée, et peut être apporté sous forme d'un cocktail d'acides aminés libres ou d'un repas complet dans lequel les protéines sont remplacées par un mélange d'acides aminés libres. La déplétion sanguine en acide aminé essentiel survient très rapidement (quelques minutes après la prise du repas carencé) et peut être très rapidement arrêtée par l'administration de l'acide aminé essentiel manquant. La carence en n'importe lequel des neuf acides aminés indispensables est fonctionnelle. Le choix de l'acide aminé (ou des acides aminés) à retirer de l'alimentation du patient pourra être fait par un homme du métier, dans un but de minimiser les effets secondaires liés au traitement et en tenant compte de la facilité pour préparer une nourriture carencée.

Il est par ailleurs possible, pour un traitement à moyen ou long terme, d'alterner la carence en différents acides aminés essentiels. Un seul repas carencé en un seul acide aminé, préférentiellement essentiel, n'étant pas toxique, l'utilisation d'une cassette selon l'invention présente un avantage unique par rapport aux autres systèmes inductibles qui ne sont pas utilisables en médecine humaine.

Dans un mode de réalisation particulier, le patient est soumis à des régimes carencés en un acide aminé successifs, chacun en un acide aminé différent (par exemple un régime carencé en leucine, suivi d'un régime carencé en valine, puis un régime carencé en lysine...). Cela permet de maintenir une carence permettant l'induction de l'expression du gène d'intérêt de l'invention, sans pour autant infliger au patient une carence prolongée en un acide aminé, qui pourrait être délétère.

Il est possible d'appliquer au patient un régime carencé en un ou plusieurs acides aminés.

L'utilisation d'une cassette de l'invention dans le cadre d'une induction de l'expression du gène par une carence en acide aminé permet de contrôler de manière précise le démarrage et la durée de la période d'induction de l'expression du gène d'intérêt. De plus, la réponse post-induction, ainsi que l'extinction de l'expression du gène d'intérêt (par administration de l'acide aminé manquant), sont rapides. Par ailleurs, la cassette d'expression utilisée selon l'invention entraine un faible niveau d'expression basale et un niveau d'activation fort.

Le patient peut également être soumis à l'administration d'une enzyme consommatrice d'acides aminés (Amino Acid Consuming Enzyme).

Un exemple d'enzyme consommatrice d'acides aminés est l'asparaginase. L'administration de ladite enzyme consommatrice d'acides aminés se fait de préférence par intraveineuse. Ce type d'administration est bien connu de l'homme de l'art (Pieters R. et al., Cancer, 2011 ; Patil S et al., Cancer Treat Rev, 2011).

Dans un autre mode particulier de réalisation, la cassette d'expression est utilisée dans des pathologies dans lesquelles la voie eIF2alpha/ATF4 est activée, tels que les cancers (Ye et al, EMBO J, 2010) ou l'épilepsie (Carnevalli et al, Biochem J, 2006). Dans ce mode de réalisation particulier, l'étape d'induction de l'expression du gène d'intérêt (ii) de la méthode de l'invention n'est plus nécessaire.

Dans ce cadre, l'invention divulgue également une méthode pour le traitement des pathologies dans lesquelles la voie eIF2alpha/ATF4 est activée par thérapie génique, ladite méthode comprenant une étape d'administration d'une cassette d'expression, d'un vecteur, d'une cellule hôte ou d'une composition pharmaceutique tels que décrits ci-dessus à un patient.

De telles pathologies comprennent notamment les cancers et l'épilepsie.

L'invention divulgue également une méthode pour le traitement des maladies par thérapie génique, ladite méthode comprenant :
(i) une étape d'administration d'une cassette d'expression, d'un vecteur, d'une cellule hôte ou d'une composition pharmaceutique tels que décrits ci-dessus à un patient, et
(ii) une étape d'induction de l'expression du gène d'intérêt compris dans ladite cassette d'expression, ledit vecteur, ladite cellule hôte ou ladite composition pharmaceutique, caractérisée en ce que ladite induction est (a) mise en oeuvre par l'application d'un régime carencé en acide aminé, préférentiellement un régime carencé en acide aminé essentiel, audit patient et (b) est simultanée, séparée ou séquentielle à l'étape (i).

Le patient peut être soumis à des régimes carencés en un acide aminé successifs, chacun en un acide aminé différent tels que décrits ci-dessus. Il est aussi possible d'appliquer au patient un régime carencé en un ou plusieurs acides aminés, préférentiellement un régime carencé en un ou plusieurs acides aminés essentiels.

La présente invention couvre enfin une combinaison comprenant:
- une cassette d'expression comprenant un gène d'intérêt codant pour un polypeptide ayant un effet thérapeutique pour une affection ciblée lié de manière opérationnelle à un promoteur inductible comprenant (i) au moins une séquence régulatrice CARE (C/EBP-ATF responsive element) contenant une séquence AARE (Amino Acid Response Element) et (ii) un promoteur minimal, un vecteur comprenant ladite cassette d'expression, une cellule hôte comprenant ledit vecteur ou ladite cassette d'expression ou une composition pharmaceutique comprenant ladite cassette d'expression, ledit vecteur ou ladite cellule hôte, et
- un régime carencé en un acide aminé essentiel,
   - pour une utilisation simultanée, séparée ou séquentielle pour le traitement par thérapie génique d'une affection ciblée choisie dans le groupe comprenant les maladies prolifératives, les maladies infectieuses, les maladies génétiques, les maladies neurologiques ou les maladies cardiovasculaires.

De préférence, le promoteur minimal utilisé dans la combinaison selon l'invention est le promoteur minimal de la thymidine kinase défini par la séquence SEQ ID NO : 1.

Dans un mode de réalisation particulier de l'invention, le promoteur minimal utilisé dans la combinaison selon l'invention est le promoteur minimal de la thymidine kinase défini par la séquence SEQ ID NO : 1 et le promoteur inductible comprend au moins une copie d'une séquence régulatrice CARE contenant une séquence AARE (Amino Acid Response Element), préférentiellement au moins deux copies d'une séquence régulatrice CARE contenant une séquence AARE (Amino Acid Response Element) choisie(s) parmi SEQ ID NO :2, SEQ ID NO : 4, SEQ ID NO :5 ou SEQ ID NO :6.

Dans un mode de réalisation préféré de l'invention, le promoteur inductible utilisé dans la combinaison selon l'invention comprend ou consiste en la séquence SEQ ID NO : 7.

### EXEMPLES

D'autres caractéristiques de l'invention apparaîtront dans les exemples qui suivent, sans pour autant que ceux-ci ne constituent une quelconque limitation de l'invention.

### Matériel et Méthodes

### Réalisation du transgène AARE-TK-LUC.

La construction 2XAARE TRIB3-TK-LUC représentée en Figure 2 a été obtenue en sous-clonant un oligonucléotide double brin contenant 2 copies de la séquence CARE/AARE de TRIB3 (-7131 à -7033) au site MluI-XhoI de la construction plasmidique TATA-TK-LUC contenant la séquence codante du gène luciférase provenant de pGL3 basic (Promega). La construction a été séquencée puis la réponse à la carence en leucine a été testée en transfection transitoire dans des cellules humaines HepG2 (Hépatome de foie) et murines (MEF : Mouse Embryonic Fibroblasts).

Le fragment d'ADN correspondant aux séquences du transgène 2XARRE-TK-LUC a ensuite été cloné aux sites BamHI/XbaI du vecteur lentiviral pRRL.PPT.SF.GFPpre 1xHS4 (Schambach A, Galla M, Maetzig T, Loew R, Baum C. Mol Ther. 2007 Jun;15(6):1167-73) contenant les séquences « insulator » 5'HS4 de la β-globine du poulet. Pour vérifier l'inductibilité du transgène AARE-LUC via la voie de signalisation eIF2α/ATF4, des cellules HeLa ont été infectées par ces particules lentivirales. Les résultats montrent que le transgène LUC, intégré de manière stable dans des cellules HeLa est induit par une diminution de la concentration de leucine de manière dose-dépendante de l'ordre de celles observées dans le plasma des souris (30 à 70 µM) et par différents concentrations de tunicamycine, un agent qui induit le stress du réticulum endoplasmique.

### Génération des souris transgéniques.

Pour générer les souris transgéniques AARE-TK-LUC, les particules lentivirales ont été microinjectées dans l'espace périvitellin d'un oocyte au stade une cellule provenant de souris C57BL/6JxDB/2J (Charles River, Wilmington, MA). Cette intégration germinale lentivirale a permis d'obtenir plus de 50% de fondateurs portant le transgène. Quinze lignées indépendantes ont été ainsi été obtenues, mais seulement 5 fondateurs comportaient au moins une copie du transgène intégré. Une descendance F1 a été obtenue pour ces 5 souris fondatrices F0 sélectionnées.

### Dosage de l'activité luciférase dans les extraits de tissus.

L'activité luciférase dans les extraits de cellules ou de tissus a été mesurée en utilisant un kit commercial (YELEN, Ensue La Redonne, France). L'activité luciférase relative correspond au rapport entre l'activité luciférase et la quantité de protéines.

### Mesure de la bioluminescence par imagerie.

L'activité luciférase a été visualisée sur les souris AARE-LUC vivantes avec une unité d'imagerie in vivo NightOWL II LB 983 NC100 (Berthold Technologies, Bad Wildbad, Allemagne). Ce système utilise une caméra CCD ultrasensible refroidie par effet Peltier à balayage lent équipée d'un objectif 25 mm/0.95 située dans une chambre noire étanche et thermocontrolée permettant la détection de niveaux très faibles de lumière émise par une cellule exprimant un gène traceur comme la luciférase. Il est également muni d'un système d'anesthésie gazeuse intégré. Pour la détection de la bioluminescence, les souris ont été anesthésiées à l'isoflurane (induction 5% isofluorane, maintien à 2% dans 70% air-30% oxygène) et ont reçu une injection intra-péritonéale d'une solution aqueuse de luciférine (Caliper LIFE SCIENCES, 150mg/kg), 10 min avant mesure de l'émission de photons (2x 4min d'intégration, pixel binning 8x8), pour obtenir une bio-distribution uniforme du substrat. Les images de bioluminescence sont présentées comme des images pseudocolorées superposées à une photographie acquise avant l'image de bioluminescence en niveau de gris en utilisant le logiciel WinLight (Berthold technologies). L'intensité du signal bioluminescent est ensuite quantifiée au niveau des régions d'intérêt avec le logiciel WinLight et exprimée en nombre de photons par seconde.

### Résultats

### Mesure de l'activité luciférase dans les tissus.

Dans un premier temps, l'évaluation de l'inductibilité du transgène AARE-TK-LUC à la carence en leucine a été réalisée en mesurant l'activité luciférase dans les différents tissus des différentes lignées transgéniques. Les souris âgées d'environ 2 mois ont été habituées pendant 7 jours à un régime contrôle (CTL) contenant tous les acides aminés indispensables. Ensuite, après avoir été mises à jeun pendant 16h, elles ont été nourries soit avec le régime CTL ou soit avec un régime carencé en leucine (-Leu) puis sacrifiées pour le prélèvement de différents tissus. L'analyse de l'activité luciférase montre que la consommation d'un régime dépourvu de leucine entraîne une forte induction de l'activité LUC dans le foie, l'intestin, le pancréas et le cerveau (Figure 3). Ce profil d'expression du gène LUC a été confirmé en mesurant le niveau des ARNm LUC.

### Mesure de la bioluminescence sur les souris vivantes.

Ensuite, l'induction de l'expression du gène de luciférase par la carence en leucine correspondant à l'activation de la voie de signalisation eIF2α/ATF4 a été visualisée par imagerie en bioluminescence sur des animaux vivants. Comme précédemment les souris ont d'abord été acclimatées avec un régime CTL pendant 7 jours et ensuite deux types d'expériences ont été réalisés:
- dans un premier cas, les souris ont été mises à jeun pendant 16h. Le jour de l'expérience, elles ont été nourries pendant 4 h, soit avec le régime CTL ou soit avec un régime carencé en leucine (-leu). Après sacrifice des souris, l'activité luciférase a été dosée dans différents tissus (figure 3).
- dans un deuxième cas, des souris mises à jeun pendant 16heures ont été nourries avec un régime carencé en leucine pendant 4h et par la suite nourries avec un régime contrôle. Cette expérience permet de mesurer la réversibilité de l'expression du transgène (Figure5). Dans ce cas, la bioluminescence provenant de la luciférase a été mesurée avant et après la consommation du régime -leu. Le comptage des photons a été réalisé au niveau de l'abdomen. Les résultats obtenus avec la face ventrale montrent clairement une forte augmentation de la luminescence dans la région abdominale 4h après le début du repas. On observe une forte diminution de la luminescence lorsque les souris sont nourries de nouveau avec un régime contrôle.

## Revendications

1. Combinaison comprenant:
- une cassette d'expression comprenant un gène d'intérêt codant pour un polypeptide ayant un effet thérapeutique pour une affection ciblée lié de manière opérationnelle à un promoteur inductible comprenant (i) au moins une séquence régulatrice CARE (C/EBP-ATF responsive élément) contenant une séquence AARE (Amino Acid Response Element) et (ii) un promoteur minimal, un vecteur comprenant ladite cassette d'expression, une cellule hôte comprenant ledit vecteur ou ladite cassette d'expression ou une composition pharmaceutique comprenant ladite cassette d'expression, ledit vecteur ou ladite cellule hôte, et
- un régime carencé en un acide aminé essentiel,
pour une utilisation simultanée, séparée ou séquentielle pour le traitement par thérapie génique d'une affection ciblée choisie dans le groupe comprenant les maladies prolifératives, les maladies infectieuses, les maladies génétiques, les maladies neurologiques ou les maladies cardiovasculaires.

2. Combinaison selon la revendication 1, **caractérisée en ce que** le promoteur minimal est le promoteur minimal de la thymidine kinase défini par la séquence SEQ ID NO : 1.

3. Combinaison selon la revendication 2, **caractérisée en ce que** le promoteur inductible comprend au moins une copie d'une séquence régulatrice CARE contenant une séquence AARE (Amino Acid Response Element), préférentiellement au moins deux copies d'une séquence régulatrice CARE contenant une séquence AARE (Amino Acid Response Element), laquelle séquence régulatrice étant la séquence SEQ ID NO: 2.

4. Combinaison selon la revendication 2, **caractérisée en ce que** le promoteur inductible comprend au moins une copie d'une séquence régulatrice CARE contenant une séquence AARE (Amino Acid Response Element), préférentiellement au moins deux copies d'une séquence régulatrice CARE contenant une séquence AARE (Amino Acid Response Element), laquelle séquence régulatrice étant la séquence SEQ ID NO: 4.

5. Combinaison selon la revendication 2, **caractérisée en ce que** le promoteur inductible comprend au moins une copie d'une séquence régulatrice CARE contenant une séquence AARE (Amino Acid Response Element), préférentiellement au moins deux copies d'une séquence régulatrice CARE contenant une séquence AARE (Amino Acid Response Element), laquelle séquence régulatrice étant la séquence SEQ ID NO : 5.

6. Combinaison selon la revendication 2, **caractérisée en ce que** le promoteur inductible comprend au moins une copie d'une séquence régulatrice CARE contenant une séquence AARE (Amino Acid Response Element), préférentiellement au moins deux copies d'une séquence régulatrice CARE contenant une séquence AARE (Amino Acid Response Element), laquelle séquence régulatrice étant la séquence SEQ ID NO : 6.

7. Combinaison selon l'une quelconque des revendications 1 à 6, **caractérisée en ce que** le promoteur inductible comprend ou consiste en la séquence SEQ ID NO : 7.

8. Cassette d'expression comprenant un gène d'intérêt lié de manière opérationnelle à un promoteur inductible comprenant (i) au moins une séquence régulatrice CARE (C/EBP-ATF responsive élément) contenant une séquence AARE (Amino Acid Response Element) et sélectionnée dans le groupe des séquences SEQ ID NO : 2, 4, 5 ou 6 et (ii) un promoteur minimal de la thymidine kinase défini par la séquence SEQ ID NO :1 pour son utilisation dans le traitement des maladies prolifératives, des maladies infectieuses, des maladies génétiques, des maladies neurologiques ou des maladies cardiovasculaires par thérapie génique.

9. Cassette d'expression pour son utilisation selon la revendication 8, **caractérisée en ce que** le promoteur inductible comprend ou consiste en la séquence SEQ ID NO : 7.

10. Vecteur comprenant une cassette d'expression comprenant un gène d'intérêt lié de manière opérationnelle à un promoteur inductible comprenant (i) au moins une séquence régulatrice CARE (C/EBP-ATF responsive élément) contenant une séquence AARE (Amino Acid Response Element) et sélectionnée dans le groupe des séquences SEQ ID NO : 2, 4, 5 ou 6 et (ii) un promoteur minimal de la thymidine kinase défini par la séquence SEQ ID NO : 1 pour son utilisation dans le traitement des maladies prolifératives, des maladies infectieuses, des maladies génétiques, des maladies neurologiques ou des maladies cardiovasculaires par thérapie génique.

11. Vecteur pour son utilisation selon la revendication 10, **caractérisé en ce que** le promoteur inductible comprend ou consiste en la séquences SEQ ID NO : 7.

12. Vecteur pour son utilisation selon la revendication 10 ou 11, **caractérisé en ce que** ledit vecteur est un vecteur lentiviral, un vecteur adénoviral ou un vecteur dérivé d'un virus associé à l'adénovirus.

13. Composition pharmaceutique comprenant une cassette d'expression comprenant un gène d'intérêt lié de manière opérationnelle à un promoteur inductible comprenant ou consistant en la séquence SEQ ID NO : 7 pour son utilisation dans le traitement des maladies prolifératives, des maladies infectieuses, des maladies génétiques, des maladies neurologiques ou des maladies cardiovasculaires par thérapie génique.

## Patentansprüche

1. Kombination, umfassend:
- eine Expressionskassette, bestehend aus einem interessierenden Gen, das auf ein Polypeptid codiert, das eine therapeutische Wirkung auf eine Zielkrankheit hat, in wirksamer Verbindung mit einem induzierbaren Promotor, bestehend aus (i) mindestens einer regulatorischen CARE-Sequenz (C/EBP-ATF responsive element), die eine AARE-Sequenz (Amino Acid Responsive Element) enthält, und (ii) einem Minimalpromotor, einen die genannte Expressionskassette enthaltenden Vektor, eine die genannte Expressionskassette oder den genannten Vektor enthaltende Wirtszelle oder eine die genannte Expressionskassette, den genannten Vektor oder die genannte Wirtszelle enthaltende pharmazeutische Zubereitung, und
- eine Diät frei von einer essentiellen Aminosäure,
zur gleichzeitigen, getrennten oder aufeinander folgenden Anwendung zur Behandlung einer aus der Gruppe, bestehend aus den Proliferationskrankheiten, den Infektionskrankheiten, den Erbkrankheiten, den neurologischen Krankheiten und den Herz-Kreislauf-Erkrankungen, ausgewählten Zielkrankheit durch Gentherapie.

2. Kombination nach Patentanspruch 1, **dadurch gekennzeichnet, dass** der Minimalpromotor der Minimalpromotor der Thymidinkinase ist, der durch die Sequenz SEQ ID NO : 1 definiert ist.

3. Kombination nach Patentanspruch 2, **dadurch gekennzeichnet, dass** der induzierbare Promotor mindestens eine Kopie einer regulatorischen CARE-Sequenz umfasst, die eine AARE-Sequenz (Amino Acid Responsive Element) enthält, vorzugsweise mindestens zwei Kopien einer regulatorischen CARE-Sequenz, die eine AARE-Sequenz (Amino Acid Responsive Element) enthält, wobei die regulatorische Sequenz die Sequenz SEQ ID NO: 2 ist.

4. Kombination nach Patentanspruch 2, **dadurch gekennzeichnet, dass** der induzierbare Promotor mindestens eine Kopie einer regulatorischen CARE-Sequenz umfasst, die eine AARE-Sequenz (Amino Acid Responsive Element) enthält, vorzugsweise mindestens zwei Kopien einer regulatorischen CARE-Sequenz, die eine AARE-Sequenz (Amino Acid Responsive Element) enthält, wobei die regulatorische Sequenz die Sequenz SEQ ID NO: 4 ist.

5. Kombination nach Patentanspruch 2, **dadurch gekennzeichnet, dass** der induzierbare Promotor mindestens eine Kopie einer regulatorischen CARE-Sequenz umfasst, die eine AARE-Sequenz (Amino Acid Responsive Element) enthält, vorzugsweise mindestens zwei Kopien einer regulatorischen CARE-Sequenz, die eine AARE-Sequenz (Amino Acid Responsive Element) enthält, wobei die regulatorische Sequenz die Sequenz SEQ ID NO : 5 ist.

6. Kombination nach Patentanspruch 2, **dadurch gekennzeichnet, dass** der induzierbare Promotor mindestens eine Kopie einer regulatorischen CARE-Sequenz umfasst, die eine AARE-Sequenz (Amino Acid Responsive Element) enthält, vorzugsweise mindestens zwei Kopien einer regulatorischen CARE-Sequenz, die eine AARE-Sequenz (Amino Acid Responsive Element) enthält, wobei die regulatorische Sequenz die Sequenz SEQ ID NO : 6 ist.

7. Kombination nach irgendeinem der Patentansprüche 1 bis 6, **dadurch gekennzeichnet, dass** der induzierbare Promotor die Sequenz SEQ ID NO : 7 umfasst oder daraus besteht.

8. Expressionskassette, bestehend aus einem interessierenden Gen in wirksamer Verbindung mit einem induzierbaren Promotor, bestehend aus (i) mindestens einer regulatorischen CARE-Sequenz (C/EBP-ATF responsive element), die eine AARE-Sequenz (Amino Acid Responsive Element) enthält, ausgewählt aus der Gruppe der Sequenzen SEQ ID NO : 2, 4, 5 und 6, und (ii) einem Minimalpromotor der Thymidinkinase, der durch die Sequenz SEQ ID NO : 1 definiert ist, zur Anwendung in der Behandlung von Proliferationskrankheiten, Infektionskrankheiten, Erbkrankheiten, neurologischen Krankheiten und Herz-Kreislauf-Erkrankungen durch Gentherapie.

9. Expressionskassette zur Anwendung nach Patentanspruch 8, **dadurch gekennzeichnet, dass** der induzierbare Promotor die Sequenz SEQ ID NO : 7 umfasst oder daraus besteht.

10. Vektor, eine Expressionskassette enthaltend, bestehend aus einem interessierenden Gen in wirksamer Verbindung mit einem induzierbaren Promotor, bestehend aus (i) mindestens einer regulatorischen CARE-Sequenz (C/EBP-ATF responsive element), die eine AARE-Sequenz (Amino Acid Responsive Element) enthält, ausgewählt aus der Gruppe der Sequenzen SEQ ID NO : 2, 4, 5 und 6, und (ii) einem Minimalpromotor der Thymidinkinase, der durch die Sequenz SEQ ID NO : 1 definiert ist, zur Anwendung in der Behandlung von Proliferationskrankheiten, Infektionskrankheiten, Erbkrankheiten, neurologischen Krankheiten und Herz-Kreislauf-Erkrankungen durch Gentherapie.

11. Vektor zur Anwendung nach Patentanspruch 10, **dadurch gekennzeichnet, dass** der induzierbare Promotor die Sequenz SEQ ID NO : 7 umfasst oder daraus besteht.

12. Vektor zur Anwendung nach Patentanspruch 10 oder 11, **dadurch gekennzeichnet, dass** der genannte Vektor ein Lentivirus-Vektor ist, ein Adenovirus-Vektor oder ein von einem einem Adenovirus zugeordneten Virus abgeleiteter Vektor.

13. Pharmazeutische Zubereitung, eine Expressionskassette enthaltend, bestehend aus einem interessierenden Gen in wirksamer Verbindung mit einem induzierbaren Promotor, der die Sequenz SEQ ID NO : 7 umfasst oder daraus besteht, zur Anwendung in der Behandlung von Proliferationskrankheiten, Infektionskrankheiten, Erbkrankheiten, neurologischen Krankheiten und Herz-Kreislauf-Erkrankungen durch Gentherapie.

## Claims

1. A combination comprising:
- An expression cassette comprising a gene of interest coding for a polypeptide having a therapeutic effect toward a targeted disease, operably linked to an inducible promotor comprising (i) at least one CARE (C/EBP-ATF responsive element) regulatory sequence comprising one AARE (Amino Acid Responsive Element) sequence, and (ii) a minimal promotor, a vector comprising said expression cassette, a host cell comprising said vector or said expression cassette, or a pharmaceutical composition comprising said expression cassette, said vector, or said host cell, and
- An essential amino-acids-deficient diet, for a simultaneous, separate, or sequential application for treating through gene therapy a targeted disease selected from the group comprising proliferative diseases, infectious diseases, genetic diseases, neurological diseases, or cardiovascular diseases.

2. The combination of claim 1, wherein the minimal promotor is the thymidine kinase minimal promotor defined by sequence SEQ ID NO: 1.

3. The combination of claim 2, wherein the inducible promotor comprises at least one copy of a CARE regulatory sequence, comprising one AARE (Amino Acid Response Element) sequence, preferably at least two copies of a CARE regulatory sequence comprising one AARE (Amino Acid Response Element) sequence, said regulatory sequence being defined by sequence SEQ ID NO: 2.

4. The combination of claim 2, wherein the inducible promotor comprises at least one copy of a CARE regulatory sequence comprising one AARE (Amino Acid Response Element) sequence, preferably at least two copies of a CARE regulatory sequence comprising one AARE (Amino Acid Response Element) sequence, said regulatory sequence being defined by sequence SEQ ID NO: 4.

5. The combination of claim 2, wherein the inducible promotor comprises at least one copy of a CARE regulatory sequence comprising an AARE (Amino Acid Response Element), preferably at least two copies of a regulatory sequence CARE comprising an AARE (Amino Acid Responsive Element) sequence, said regulatory sequence is defined by sequence SEQ ID NO: 5.

6. The combination of claim 2, wherein the inducible promotor comprises at least one copy of a CARE regulatory sequence comprising an AARE (Amino Acid Response Element) sequence, preferably at least two copies of a CARE regulatory sequence comprising one AARE (Amino Acid Responsive Element) sequence, said regulatory sequence is defined by sequence SEQ ID NO: 6.

7. The combination of anyone of claims 1-6, wherein inducible promotor comprises or consists in sequence SEQ ID NO: 7.

8. An expression cassette comprising a gene of interest operably linked to an inducible promotor comprising (i) at least one CARE (C/EBP-ATF responsive element) regulatory sequence comprising one AARE (Amino Acid Response Element) sequence and selected in the group comprising SEQ ID NO: 2, 4, 5, or 6 and (ii) a thymidine kinase minimal promotor defined by sequence SEQ ID NO: 1 for use in the treatment of proliferative diseases, infectious diseases, genetic diseases, neurological diseases or cardiovascular diseases through gene therapy.

9. The expression cassette for use according to claim 8, wherein inducible promotor comprises or consists in sequence SEQ ID NO: 7.

10. A vector comprising an expression cassette comprising a gene of interest operably linked to an inducible promotor comprising (i) at least one CARE (C/EBP-ATF responsive element) regulatory sequence comprising at least one AARE (Amino Acid Response Element) sequence and selected in the group comprising sequences SEQ ID NO: 2, 4, 5 or 6 and (ii) a thymidine kinase minimal promotor defined by sequence SEQ ID NO: 1 for use in the treatment of proliferative diseases, infectious diseases, genetic diseases, neurological diseases or cardiovascular diseases through gene therapy.

11. The vector for use according to claim 10, wherein the inducible promotor comprises or consisting in sequence SEQ ID NO: 7.

12. The vector for use according to claims 10-11, wherein said vector is a lentiviral vector, an adenoviral vector or a vector derived from an adenovirus-associated virus.

13. A pharmaceutical composition comprising an expression cassette comprising a gene of interest operably linked to an inducible promotor that comprises or consists in sequence SEQ ID NO: 7 for use in the treatment of proliferative diseases, infectious diseases, genetic diseases, neurological diseases or cardiovascular diseases through gene therapy.


### SEQUENCE LISTING

<110> Institut National de Recherche Agronomique
<120> CASSETTE D'EXPRESSION INDUCTIBLE ET SES UTILISATIONS
<130> INR-B-0009
<150> FR 11 03392
   <151> 2011-11-08
<160> 8
<170> PatentIn version 3.5
<210> 1
   <211> 89
   <212> DNA
   <213> Human Herpes virus type 1
<400> 1
   gtccacttcg catattaagg tgacgcgtgt ggcctcgaac accgagcgac cctgcagcga 60
   cccgcttaac agcgtcaaca gcgtgccgc 89
<210> 2
   <211> 16
   <212> DNA
   <213> Homo sapiens
<400> 2
   cggtttgcat cacccg 16
<210> 3
   <211> 16
   <212> DNA
   <213> Homo sapiens
<400> 3
   aacattgcat catccc 16
<210> 4
   <211> 16
   <212> DNA
   <213> Homo sapiens
<400> 4
   gaagtttcat catgcc 16
<210> 5
   <211> 16
   <212> DNA
   <213> Homo sapiens
<400> 5
   agcgttgcat cacccc 16
<210> 6
   <211> 16
   <212> DNA
   <213> Homo sapiens
<400> 6
   gatattgcat cagttt 16
<210> 7
   <211> 311
   <212> DNA
   <213> Artificial Sequence
<220>
   <223> Transgene
<400> 7
   gattagctcc ggtttgcatc acccggaccg ggggattagc tccggtttgc atcacccgga 60
   ccgggggatt agctccggtt tgcatcaccc ggaccggggg ccgggcgcgt gctagcgatt 120
   agctccggtt tgcatcaccc ggaccggggg attagctccg gtttgcatca cccggaccgg 180
   gggattagct ccggtttgca tcacccggac cggggactcg aggtccactt cgcatattaa 240
   ggtgacgcgt gtggcctcga acaccgagcg accctgcagc gacccgctta acagcgtcaa 300
   cagcgtgccg c 311
<210> 8
   <211> 300
   <212> DNA
   <213> Homo sapiens
<400> 8
   tttctgcttg ctgtctgccg gtttaagttg tgtgctcggg tgtccctttc ctcttcccct 60
   cccgcagggc ttgcggccac catggcgtat tagaggcagc agtgcctgcg gcagcgttgg 120
   cctttgcagc ggcggcagca gcaccaggct ctgcagcggc aacccccacc ggcctaagcc 180
   atggcgctct tcacgaaatc cagcagcagt gttgctgtaa cggacaaaga taccttcgag 240
   ttaagcacat tcctcgaatc cagcaaagcc ccacaacatg accgagatga gcttcctgaa 300